# FASCICULE DE BREVET EUROPEEN

(11) **EP 1 990 043 B1**
(45) Date de publication et mention de la délivrance du brevet: **29.06.2016**
(21) Numéro de dépôt: 08155494.1
(22) Date de dépôt: 30.04.2008
(51) Int. Cl.: A61K 8/898, A61Q 5/10

(54) **Composition pour la teinture des fibres kératiniques comprenant au moins une silicone aminée particulière et de la monoéthanolamine**
Zusammensetzung zum Färben von Keratinfasern, die mindestens ein spezielles Aminosilikon und Monoethanolamin umfasst
Composition for dyeing keratinous fibres comprising at least one specific amine silicone and monoethanolamine.

(30) Priorité: 07.05.2007 FR 0754911
(43) Date de publication de la demande: 12.11.2008
(73) Titulaire: L'Oréal, 75008 Paris (FR)
(72) Inventeur: Audousset, Marie-Pascale, 92600 Asnières (FR); Rondeau, Christine, 78500 Sartrouville (FR)
(74) Mandataire: Zapalowicz, Francis

(56) Documents cités:
- EP-A- 0 497 163
- EP-A- 0 890 355
- EP-A- 1 426 038
- EP-A- 1 803 434
- US-A1- 2002 189 034

## Description

La présente invention concerne une composition pour la teinture des fibres kératiniques, en particulier des fibres kératiniques humaines telles que les cheveux, comprenant au moins une base d'oxydation, au moins un coupleur, au moins une silicone aminée particulière et au moins de la monoéthanolamine. L'invention porte également sur un procédé de teinture des fibres kératiniques mettant en oeuvre cette composition ainsi qu'un dispositif la contenant.

Il est connu de teindre les fibres kératiniques et en particulier les cheveux humains avec des compositions tinctoriales contenant des précurseurs de colorants d'oxydation, appelés généralement bases d'oxydation, tels que les ortho ou paraphénylènediamines, les ortho ou paraaminophénols et les bases hétérocycliques. Les précurseurs de colorants d'oxydation ou bases d'oxydation sont initialement des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les méta-diaminobenzènes, les méta-aminophénols, les métadiphénols et certains composés hétérocycliques tels que des composés indoliques.

La variété des molécules mises en jeu au niveau des bases d'oxydation et des coupleurs permet l'obtention d'une riche palette de couleurs.

Cependant, les colorations d'oxydation classiques peuvent entraîner des désagréments au moment de l'application tels que des rougeurs ou des picotements.

EP1426038 décrit des compositions de teinture d'oxydation comprenant une silicone aminée et un polymère associatif. L"exemple décrit des compositions comprenant de l'ammoniaque dans une teneur de 11 % en pois et de la monoéthanolamine à une concentration de 1 % avant mélange avec l'agent oxydant. But de ce document est de proposer des compositions capables de conférer de bonnes propriétés cosmétiques.

Il existe donc un réel besoin de trouver des compositions tinctoriales qui permettent de minimiser les irritations du cuir chevelu afin de rendre l'application de ces compositions plus confortable pour l'utilisateur tout en conduisant à des colorations satisfaisantes.

De manière surprenante et avantageuse, la Demanderesse vient de découvrir qu'il est possible d'obtenir de nouvelles compositions de teintures, capables de réduire l'inconfort pouvant être ressenti au moment de l'application de la composition de teinture qui peut se traduire par l'apparition de rougeurs et/ou de picotement au niveau du cuir chevelu tout en conduisant à des colorations puissantes, chromatiques, peu sélectives et résistant bien aux diverses agressions que peuvent subir les cheveux.

La présente invention a donc notamment pour objet une composition pour la teinture des fibres kératiniques, en particulier des fibres kératiniques humaines telles que les cheveux, comprenant au moins une base d'oxydation, au moins un coupleur, un agent oxydant, de la monoéthanolamine présente en une quantité allant de 1 à 10% en poids, par rapport au poids total de la composition, et au moins une silicone aminée de masse moléculaire en poids supérieure à 75000.

Un autre objet de la présente invention consiste en un procédé de teinture des fibres kératiniques humaines et plus particulièrement des cheveux, consistant à appliquer sur les fibres la composition selon l'invention.

D'autres objets, caractéristiques, aspects et avantages de l'invention apparaîtront encore plus clairement à la lecture de la description et des exemples qui suivent.

La composition tinctoriale conforme à l'invention permet de minimiser l'inconfort pouvant être ressenti au moment de l'application de la composition de teinture.

La composition tinctoriale conforme à l'invention permet également d'aboutir à des colorations puissantes, chromatiques, présentant une faible sélectivité et d'excellentes propriétés de résistances notamment vis-à-vis des shampooings.

Les silicones aminées utilisées dans la composition conforme à l'invention présentent la formule générale suivante : 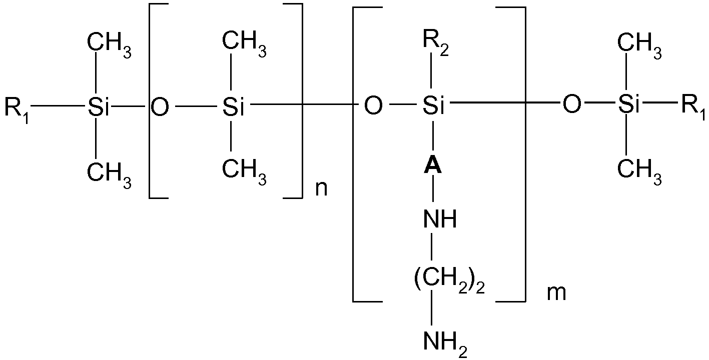 dans laquelle :
A désigne un radical alkylène linéaire ou ramifié en C₂-C₈ et de préférence en C₃.
R₁ et R₂ désignent indépendamment l'un de l'autre un radical alkyle en C₁-C₄, de préférence méthyle ou un radical alcoxy en C₁-C₄ de préférence méthoxy ou un radical hydroxy, m et n sont des nombres tels que la masse moléculaire en poids (M_{W}) soit supérieure ou égale à 75 000.

De préférence la viscosité de la silicone aminée est supérieure à 25 000 mm²/s mesurée à 25°C.

Plus préférentiellement, la viscosité de la silicone aminée est comprise entre 30 000 et 200 000 mm²/s à 25°C et encore plus préférentiellement entre 30 000 et 150 000 mm²/s mesurée à 25°C. De préférence, la charge cationique de la silicone aminée est inférieure ou égale à 0,06 meq/g.

Les viscosités des silicones sont par exemple mesurées selon la norme « ASTM 445 Appendice C ».

De préférence, la silicone aminée présente une masse moléculaire moyenne en poids allant de 75000 à 1 000 000 et encore plus préférentiellement allant de 100 000 à 200 000.

Les masses moléculaires moyennes en poids de ces silicones aminées sont mesurées par Chromatographie par Perméation de Gel (GPC) à température ambiante en équivalent polystyrène. Les colonnes utilisées sont des colonnes µ styragel. L'éluant est le THF, le débit est de 1 ml/mn. On injecte 200 µl d'une solution à 0,5% en poids de silicone dans le THF. La détection se fait par réfractométrie et UVmétrie.

Lorsque ces silicones aminées sont mises en oeuvre, une forme de réalisation particulièrement intéressante est leur utilisation sous forme d'émulsion huile-dans-eau.

L'émulsion huile-dans-eau peut comprendre un ou plusieurs tensioactifs. Les tensioactifs peuvent être de toute nature mais de préférence cationique et/ou non ionique.

Les particules de silicone dans l'émulsion ont une taille moyenne allant généralement de 3 nm à 500 nanomètres, de préférence de 5 nm à 300 nanomètres, plus particulièrement de 10 nm à 275 nanomètres et encore plus particulièrement de 150 nm à 275 nanomètres.

Une silicone aminée particulièrement préférée répondant à cette formule est par exemple la DC2-8299® de la société DOW CORNING.

La ou les silicones aminées est ou sont utilisées dans la composition tinctoriale selon l'invention en une quantité allant de 0,01 à 20 % en poids, de préférence en une quantité allant de 0,1 à 15 % en poids, et encore plus préférentiellement en une quantité allant de 0,5 à 10 % en poids, par rapport au poids total de la composition.

Les bases d'oxydation utilisables dans le cadre de la présente invention sont choisies parmi celles classiquement connues en teinture d'oxydation, et parmi lesquelles on peut notamment citer les ortho- et para-phénylènediamines, les bases doubles, les ortho- et para-aminophénols, les bases hétérocycliques suivantes ainsi que leurs sels d'addition avec un acide.

On peut notamment citer :
(A) les paraphénylènediamines de formule (II) suivante et leurs sels d'addition avec un acide : 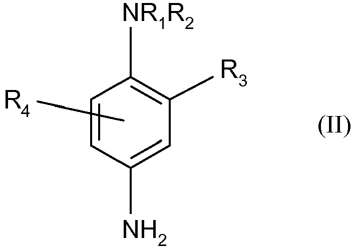 dans laquelle :
   R₁ représente un atome d'hydrogène, un radical alkyle en C₁-C₄, monohydroxyalkyle en C₁-C₄, polyhydroxyalkyle en C₂-C₄ alcoxy(C₁-C₄)alkyle(C₁-C₄), alkyle en C₁-C₄ substitué par un groupement azoté, phényle ou 4'-aminophényle ;
   R₂ représente un atome d'hydrogène, un radical alkyle en C₁-C₄, monohydroxyalkyle en C₁-C₄ ou polyhydroxyalkyle en C₂-C₄, alcoxy(C₁-C₄)alkyle(C₁-C₄) ou alkyle en C₁-C₄ substitué par un groupement azoté ; R₁ et R₂ peuvent également former avec l'atome d'azote qui les porte un hétérocycle azoté à 5 ou 6 chaînons éventuellement substitué par un ou plusieurs groupements alkyle, hydroxy ou uréido;
   R₃ représente un atome d'hydrogène, un atome d'halogène tel qu'un atome de chlore, un radical alkyle en C₁-C₄, sulfo, carboxy, monohydroxyalkyle en C₁-C₄ ou hydroxyalcoxy en C₁-C₄, acétylaminoalcoxy en C₁-C₄, mésylaminoalcoxy en C₁-C₄ ou carbamoylaminoalcoxy en C₁-C₄ ;
   R₄ représente un atome d'hydrogène, d'halogène ou un radical alkyle en C₁-C₄.

   Parmi les groupements azotés de la formule (II) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.
   Parmi les paraphénylènediamines de formule (II) ci-dessus, on peut plus particulièrement citer la paraphénylènediamine, la paratoluylènediamine, la 2-chloro-paraphénylènediamine, la 2,3-diméthyl-paraphénylènediamine, la 2,6-diméthyl-paraphénylènediamine, la 2,6-diéthyl-paraphénylènediamine, la 2,5-diméthyl-paraphénylènediamine, la N,N-diméthyl-paraphénylènediamine, la N,N-diéthyl-paraphénylènediamine, la N,N-dipropyl-paraphénylènediamine, la 4-amino-N,N-diéthyl-3-méthyl-aniline, la N,N-bis-(β-hydroxyéthyl)-paraphénylènediamine, la 4-N,N-bis-(β-hydroxyéthyl)amino-2-méthyl-aniline, la 4-N,N-bis-(β-hydroxyéthyl)-amino 2-chloro-aniline, la 2-β-hydroxyéthyl-paraphénylènediamine, la 2-fluoro-paraphénylènediamine, la 2-isopropyl-paraphénylènediamine, la N-(β-hydroxypropyl)-paraphénylènediamine, la 2-hydroxyméthyl-paraphénylènediamine, la N,N-diméthyl-3-méthyl-paraphénylènediamine, la N,N-(éthyl,β-hydroxyéthyl)-paraphénylènediamine, la N-(β,γ-dihydroxypropyl)-paraphénylènediamine, la N-(4'-aminophényl)-paraphénylènediamine, la N-phényl-paraphénylènediamine, la 2-β-hydroxyéthyloxy-paraphénylènediamine, la 2-β-acétylaminoéthyloxy-paraphénylènediamine, la N-(β-méthoxyéthyl)-paraphénylènediamine, 2-méthyl-1-N-β-hydroxyéthyl-paraphénylènediamine, et leurs sels d'addition avec un acide.
   Parmi les paraphénylènediamines de formule (II) ci-dessus, on préfère tout particulièrement la paraphénylènediamine, la paratoluylènediamine, la 2-isopropyl-paraphénylènediamine, la 2-β-hydroxyéthyl-paraphénylènediamine, la 2-β-hydroxyéthyloxy-paraphénylènediamine, la 2,6-diméthyl-paraphénylène-diamine, la 2,6-diéthyl-paraphénylènediamine, la 2,3-diméthyl-paraphénylènediamine, la N,N-bis-(β-hydroxyéthyl)-paraphénylènediamine, la 2-chloro-paraphénylènediamine, et leurs sels d'addition avec un acide.
-(B) Selon l'invention, on entend par bases doubles, les composés comportant au moins deux noyaux aromatiques sur lesquels sont portés des groupements amino et/ou hydroxyle.
   Parmi les bases doubles utilisables à titre de bases d'oxydation dans les compositions tinctoriales prêtes à l'emploi conformes à l'invention, on peut notamment citer les composés répondant à la formule (III) suivante, et leurs sels d'addition avec un acide : 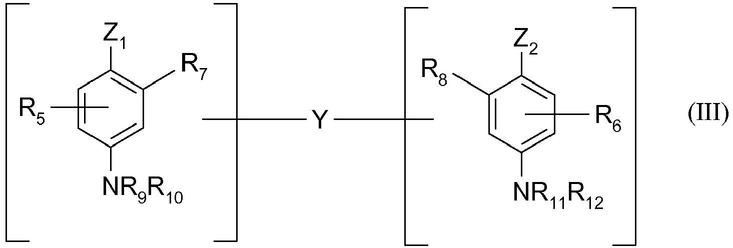 dans laquelle :
   - Z₁ et Z₂, identiques ou différents, représentent un radical hydroxyle ou
   - NH₂ pouvant être substitué par un radical alkyle en C₁-C₄ ou par un bras de liaison Y ;
   - le bras de liaison Y représente une chaîne alkylène comportant de 1 à 14 atomes de carbone, linéaire ou ramifiée pouvant être interrompue ou terminée par un ou plusieurs groupements azotés et/ou par un ou plusieurs hétéroatomes tels que des atomes d'oxygène, de soufre ou d'azote, et éventuellement substituée par un ou plusieurs radicaux hydroxyle ou alcoxy en C₁-C₆ ;
   - R₅ et R₆ représentent un atome d'hydrogène ou d'halogène, un radical alkyle en C₁-C₄, monohydroxyalkyle en C₁-C₄, polyhydroxyalkyle en C₂-C₄, aminoalkyle en C₁-C₄ ou un bras de liaison Y ;
   - R₇, R₈, R₉, R₁₀, R₁₁ et R₁₂, identiques ou différents, représentent un atome d'hydrogène, un bras de liaison Y ou un radical alkyle en C₁-C₄ ; étant entendu que les composés de formule (III) ne comportent qu'un seul bras de liaison Y par molécule.

   Parmi les groupements azotés de la formule (III) ci-dessus, on peut citer notamment les radicaux amino, monoalkyl(C₁-C₄)amino, dialkyl(C₁-C₄)amino, trialkyl(C₁-C₄)amino, monohydroxyalkyl(C₁-C₄)amino, imidazolinium et ammonium.
   Parmi les bases doubles de formules (III) ci-dessus, on peut plus particulièrement citer le N,N'-bis-(β-hydroxyéthyl)-N,N'-bis-(4'-aminophényl)-1,3-diamino-propanol, la N,N'-bis-(β-hydroxyéthyl)-N,N'-bis-(4'-aminophényl)-éthylènediamine, la N,N'-bis-(4-aminophényl)-tétraméthylènediamine, la N,N'-bis-(β-hydroxyéthyl)-N,N'-bis-(4-aminophényl)-tétraméthylènediamine, la N,N'-bis-(4-méthyl-aminophényl)-tétraméthylènediamine, la N,N'-bis-(éthyl)-N,N'-bis-(4'-amino-3'-méthylphényl)-éthylènediamine, le 1,8-bis-(2,5-diaminophénoxy)-3,5-dioxaoctane, et leurs sels d'addition avec un acide.
   Parmi ces bases doubles de formule (III), le N,N'-bis-(β-hydroxyéthyl)-N,N'-bis-(4'-aminophényl)-1,3-diamino-propanol, le 1,8-bis-(2,5-diaminophénoxy)-3,5-dioxaoctane ou l'un de leurs sels d'addition avec un acide sont particulièrement préférés.
- (C) les para-aminophénols répondant à la formule (IV) suivante, et leurs sels d'addition avec un acide : 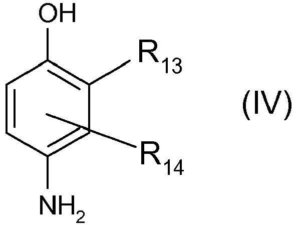 dans laquelle :
   R₁₃ représente un atome d'hydrogène, un atome d'halogène tel que le fluor, un radical alkyle en C₁-C₄, monohydroxyalkyle en C₁-C₄, alcoxy(C₁-C₄)alkyle(C₁-C₄) ou aminoalkyle en C₁-C₄, ou hydroxyalkyl(C₁-C₄)aminoalkyle en C₁-C₄.
   R₁₄ représente un atome d'hydrogène ou un atome d'halogène tel que le fluor, un radical alkyle en C₁-C₄, monohydroxyalkyle en C₁-C₄, polyhydroxyalkyle en C₂-C₄, aminoalkyle en C₁-C₄, cyanoalkyle en C₁-C₄ ou alcoxy(C₁-C₄)alkyle(C₁-C₄).

   Parmi les para-aminophénols de formule (IV) ci-dessus, on peut plus particulièrement citer le para-aminophénol, le 4-amino-3-méthyl-phénol, le 4-amino-3-fluoro-phénol, le 4-amino-3-hydroxyméthyl-phénol, le 4-amino-2-méthyl-phénol, le 4-amino-2-hydroxyméthyl-phénol, le 4-amino-2-méthoxyméthyl-phénol, le 4-amino-2-aminométhyl-phénol, le 4-amino-2-(β-hydroxyéthyl-aminométhyl)-phénol, et leurs sels d'addition avec un acide.
-(D) les ortho-aminophénols utilisables à titre de bases d'oxydation dans le cadre de la présente l'invention, sont notamment choisis parmi le 2-amino-phénol, le 2-amino-1-hydroxy-5-méthyl-benzène, le 2-amino-1-hydroxy-6-méthyl-benzène, le 5-acétamido-2-amino-phénol, et leurs sels d'addition avec un acide.
-(E) parmi les bases hétérocycliques utilisables à titre de bases d'oxydation dans les compositions tinctoriales conformes à l'invention, on peut plus particulièrement citer les dérivés pyridiniques, les dérivés pyrimidiniques, les dérivés pyrazoliques, et leurs sels d'addition avec un acide.

Parmi les dérivés pyridiniques, on peut plus particulièrement citer les composés décrits par exemple dans les brevets GB 1 026 978 et GB 1 153 196, comme la 2,5-diamino-pyridine, la 2-(4-méthoxyphényl)amino-3-amino-pyridine, la 2,3-diamino-6-méthoxy-pyridine, la 2-(β-méthoxyéthyl)amino-3-amino-6-méthoxy pyridine, la 3,4-diamino-pyridine, et leurs sels d'addition avec un acide.

Parmi les dérivés pyrimidiniques, on peut plus particulièrement citer les composés décrits par exemple dans les brevets allemand DE 2 359 399 ou japonais JP 88-169 571 et JP 91-10659 ou demandes de brevet WO 96/15765, comme la 2,4,5,6-tétra-aminopyrimidine, la 4-hydroxy-2,5,6-triaminopyrimidine, la 2-hydroxy-4,5,6-triaminopyrimidine, la 2,4-dihydroxy-5,6-diaminopyrimidine, la 2,5,6-triaminopyrimidine, et les dérivés pyrazolo-pyrimidiniques tels ceux mentionnés dans la demande de brevet FR-A-2 750 048 et parmi lesquels on peut citer la pyrazolo-[1,5-a]-pyrimidine-3,7-diamine ; la 2,5-diméthyl-pyrazolo-[1,5-a]-pyrimidine-3,7-diamine ; la pyrazolo-[1,5-a]-pyrimidine-3,5-diamine ; la 2,7-diméthyl-pyrazolo-[1,5-a]-pyrimidine-3,5-diamine ; le 3-amino-pyrazolo-[1,5-a]-pyrimidin-7-ol ; le 3-amino-pyrazolo-[1,5-a]-pyrimidin-5-ol ; le 2-(3-amino pyrazolo-[1,5-a]-pyrimidin-7-ylamino)-éthanol; le 2-(7-amino-pyrazolo-[1,5-a]-pyrimidin-3-ylamino)-éthanol; le 2-[(3-amino-pyrazolo[1,5-a]pyrimidin-7-yl)-(2-hydroxy-éthyl)-amino]-éthanol; le 2-[(7-amino-pyrazolo[1,5-a]pyrimidin-3-yl)-(2-hydroxy-éthyl)-amino]-éthanol; la 5,6-diméthyl-pyrazolo-[1,5-a]-pyrimidine-3,7-diamine; la 2,6-diméthyl-pyrazolo-[1,5-a]-pyrimidine-3,7-diamine; la 2, 5, N7, N7-tetraméthyl-pyrazolo-[1,5-a]-pyrimidine-3,7-diamine; la 3-amino-5-méthyl-7-imidazolylpropylamino-pyrazolo-[1,5-a]-pyrimidine; et leurs sels d'addition et leurs formes tautomères, lorsqu'il existe un équilibre tautomérique et leurs sels d'addition avec un acide.

Parmi les dérivés pyrazoliques, on peut plus particulièrement citer les composés décrits dans les brevets DE 3 843 892, DE 4 133 957 et demandes de brevet WO 94/08969, WO 94/08970, FR-A-2 733 749 et DE 195 43 988 comme le 4,5-diamino-1-méthyl-pyrazole, le 3,4-diamino-pyrazole, le 4,5-diamino-1-(4'-chlorobenzyl)-pyrazole, le 4,5-diamino 1,3-diméthyl-pyrazole, le 4,5-diamino-3-méthyl-1-phényl-pyrazole, le 4,5-diamino 1-méthyl-3-phényl-pyrazole, le 4-amino-1,3-diméthyl-5-hydrazino-pyrazole, le 1-benzyl-4,5-diamino-3-méthyl-pyrazole, le 4,5-diamino-3-tert-butyl-1-méthyl-pyrazole, le 4,5-diamino-1-tert-butyl-3-méthyl-pyrazole, le 4,5-diamino-1-(β-hydroxyéthyl)-3-méthyl pyrazole, le 4,5-diamino-1-(β-hydroxyéthyl)-pyrazole, le 4,5-diamino-1-éthyl-3-méthyl-pyrazole, le 4,5-diamino-1-éthyl-3-(4'-méthoxyphényl)-pyrazole, le 4,5-diamino-1-éthyl-3-hydroxyméthyl-pyrazole, le 4,5-diamino-3-hydroxyméthyl-1-méthyl-pyrazole, le 4,5-diamino-3-hydroxyméthyl-1-isopropyl-pyrazole, le 4,5-diamino-3-méthyl-1-isopropyl-pyrazole, le 4-amino-5-(2'-aminoéthyl)amino-1,3-diméthyl-pyrazole, le 3,4,5-triamino-pyrazole, le 1-méthyl-3,4,5-triamino-pyrazole, le 3,5-diamino-1-méthyl-4-méthylamino-pyrazole, le 3,5-diamino-4-(β-hydroxyéthyl)amino-1-méthyl-pyrazole, et leurs sels d'addition avec un acide.

Les ou les coupleurs utilisables dans la composition prêtre à l'emploi selon l'invention sont ceux classiquement utilisés dans les compositions de teinture d'oxydation, c'est-à-dire les méta-aminophénols, les méta-phénylènediamines, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et leurs sels d'addition avec un acide.

Selon un mode de réalisation préféré, le ou les coupleurs est ou sont choisis parmi les métaphénylènediamines, les méta-aminophénols, les métadiphénols, les coupleurs hétérocycliques, et les sels d'addition de ces composés avec un acide.

Ces coupleurs sont plus particulièrement choisis parmi le 2,4-diamino 1-(β-hydroxyéthyloxy)-benzène, le 2-méthyl-5-amino-phénol, le 5-N-(β-hydroxyéthyl)amino-2-méthyl-phénol, le 3-amino-phénol, le 1,3-dihydroxy-benzène, le 1,3-dihydroxy-2-méthyl-benzène, le 4-chloro-1,3-dihydroxy-benzène, le 2-amino 4-(β-hydroxyéthylamino)-1-méthoxy-benzène, le 1,3-diamino-benzène, le 1,3-bis-(2,4-diaminophénoxy)-propane, le sésamol, le 1-amino-2-méthoxy-4,5-méthylènedioxy benzène, l'α-naphtol, le 6-hydroxy-indole, le 4-hydroxy-indole, le 4-hydroxy-N-méthyl indole, la 6-hydroxy-indoline, la 2,6-dihydroxy-4-méthyl-pyridine, le 1-H-3-méthyl-pyrazole-5-one, le 1-phényl-3-méthyl-pyrazole-5-one, la 2-amino-3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

La ou les bases d'oxydation et le ou les coupleurs sont en général présentes en une quantité allant de 0,0005 à 12 % en poids, de préférence en une quantité allant de 0,01 à 8 % en poids, par rapport au poids total de la composition.

D'une manière générale, les sels d'addition avec un acide des bases d'oxydation et des coupleurs sont notamment choisis parmi les chlorhydrates, les bromhydrates, les sulfates et les tartrates, les lactates et les acétates.

Selon un mode de réalisation particulier, la composition de l'invention contient en outre du métasilicate de sodium.

Lorsqu'il est présent, le métasilicate de sodium représente une quantité allant de 0,1 à 10 % en poids, de préférence en une quantité allant de 0,5 à 5 % en poids, et encore plus préférentiellement en une quantité allant de 1 à 4 % en poids, par rapport au poids total de ladite composition.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiants.

Les adjuvants ci dessus sont en général présents en quantité comprise pour chacun d'eux entre 0,01 et 20 % en poids par rapport au poids de la composition.

Bien entendu, l'homme de l'art veillera à choisir le ou les éventuels composés complémentaires mentionnés ci-avant, de manière telle que les propriétés avantageuses attachées intrinsèquement à la composition tinctoriale selon l'invention ne soient pas, ou substantiellement pas, altérées par la ou les adjonctions envisagées.

Le milieu approprié pour la teinture (ou support) de la composition tinctoriale prête à l'emploi conforme à l'invention est de préférence un milieu aqueux constitué par de l'eau et peut avantageusement contenir des solvants organiques acceptables sur le plan cosmétique, dont plus particulièrement, des alcools tels que l'alcool éthylique, l'alcool isopropylique, l'alcool benzylique, et l'alcool phényléthylique, ou des glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol. Les solvants peuvent alors être présents dans des concentrations comprises entre environ 0,5 et 20% en poids et, de préférence, entre environ 2 et 10% en poids par rapport au poids total de la composition.

Le pH de la composition est généralement compris entre les valeurs 3 et 12. Il est de préférence compris entre 7 et 11, et peut être ajusté à la valeur désirée au moyen de la monoéthanolamine et d'agents acidifiants ou alcalinisants bien connus de l'état de la technique en teinture des fibres kératiniques.

En tant qu'agent alcalinisant, la monoéthanolamine est préférée bien qu'elle puisse être associée à d'autres agents alcalinisants classique. Selon un mode de réalisation particulier, la composition ne contient que la monoéthanolamine en tant qu'agent alcalinisant.

Parmi les agents acidifiants on peut citer, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

La présente invention concerne aussi un procédé de teinture des fibres kératiniques, en particulier des fibres kératiniques humaines telles que les cheveux, consistant à appliquer sur lesdites fibres une composition telle que définie ci-dessus, en présence d'un agent oxydant pendant un temps suffisant pour développer la coloration désirée.

Selon ce procédé, on applique sur les fibres au moins une composition tinctoriale telle que définie précédemment, pendant un temps suffisant pour développer la coloration désirée, après quoi on rince, on lave éventuellement au shampooing, on rince à nouveau et on sèche.

Selon un mode de réalisation particulier, l'agent oxydant peut être ajouté à la composition de l'invention directement sur les fibres kératiniques avant ou après l'application de la composition de la présente invention. Selon un mode de réalisation particulier, l'agent oxydant est ajouté à la composition de l'invention au moment de l'emploi.

Le mélange obtenu est ensuite appliqué sur les fibres kératiniques. Après un temps de pose de 3 à 50 minutes environ, de préférence 5 à 30 minutes environ, les fibres kératiniques sont rincées, lavées au shampooing, rincées à nouveau puis séchées.

Les agents oxydants classiquement utilisés pour la teinture d'oxydation des fibres kératiniques sont par exemple le peroxyde d'hydrogène, le peroxyde d'urée, les bromates de métaux alcalins, les persels tels que les perborates et persulfates, les peracides et les enzymes oxydases parmi lesquelles on peut citer les peroxydases, les oxydo-réductases à 2 électrons telles que les uricases et les oxygénases à 4 électrons comme les laccases. Le peroxyde d'hydrogène est particulièrement préféré.

La composition oxydante peut également renfermer divers adjuvants utilisés classiquement dans les compositions pour la teinture des cheveux et tels que définis précédemment.

Selon un mode de réalisation particulier, la concentration de monoéthanolamine dans la composition après mélange avec l'agent oxydant est comprise entre 0,5 et 10 en poids, plus particulièrement entre 1 et 5% en poids, par rapport au poids total de la composition résultante du mélange.

L'invention concerne également l'utilisation de la composition telle que définie précédemment pour la teinture des fibres kératiniques, en particulier des fibres kératiniques humaines telles que les cheveux.

L'invention concerne également un dispositif à plusieurs compartiments ou « kit » pour la teinture ou tout autre système de conditionnement à plusieurs compartiments dont un premier compartiment renferme la composition de l'invention telle que définie ci-dessus, et un autre compartiment renfermant une composition contenant un ou plusieurs agents oxydants.

Ces dispositifs peuvent être équipés d'un moyen permettant de délivrer sur les cheveux le mélange souhaité, tel que les dispositifs décrits dans le brevet FR-2856913.

Les exemples qui suivent sont destinés à illustrer l'invention sans pour autant en limiter la portée.

### EXEMPLE

### Exemple I

On a préparé les deux compositions tinctoriales conformes à l'invention à partir des composés suivants:
(exprimé en grammes sauf autre indication)

| | Exemple 1 | Exemple 2 |
|---|---|---|
| 1-Méthyl-2,5-diamino-benzène | 1,7 | 0,5 |
| 1-Hydroxy-4amino-benzène | | 0,4 |
| 1,3-Dihydroxybenzène | 1 | 0,25 |
| 1-Hydroxy-3-amino-benzène | 0,07 | |
| Dichlorydrate de 1-β-hydroxyéthyloxy - 2,4-diamino-benzène | 0,03 | |
| 2-Méthyl-1,3-dihydroxybenzène | 0,5 | 0,3 |
| 1-méthyl-2-hydroxy-4-amino-benzène | | 0,25 |
| 1-méthyl-2-hydroxy-4-β-hydroxyéthylamino-benzène | | 0,05 |
| 6-hydroxyindole | | 0,01 |
| Métasilicate de sodium anhydre | | 2 |
| Monoéthanolamine pure | 5 | 10 |
| Polyquaternium 6 : vendu sous la dénomination Merquat 100 par Ondeo | | 3 |
| Polyquaternium 22 : vendu sous la dénomination Merquat 280 par Ondeo | 1,5 | |
| Chlorure Hexadimethrine : vendu sous la dénomination Mexomere PO par Chimex | 1,5 | |
| Propylène glycol | 10 | 10 |
| Acide polyacrylique réticulé | 0,4 | 0,4 |
| Alcool laurique oxyethylène à 12 moles d'oxyde d'éthylène | 7,5 | 7,5 |
| Alcool oleocétylique oxyéthylène à 30 moles d'oxyde d'éthylène | 6 | 6 |
| Alcool décylique oxyéthylène à 3 moles d'oxyde d'éthylène | 8 | 8 |
| Acide laurique | 2,5 | 2,5 |
| Alcool cétylstéarylique 50/50 | 10 | 10 |
| Silice pyrogénée | 1 | 1 |
| DC2-8299® de la société DOW CORNING) (silicone aminée de l'invention). | 3 % MA | 1 % MA |
| Monostéarate de glycérol | 1 | 1 |
| Réducteur, séquestrant, antioxydant, parfum | qs | qs |
| Eau déminéralisée qs | 100 | 100 |

On a mélangé une partie de chacune de ces compositions, avec une partie et demie de peroxyde d'hydrogène en solution aqueuse. Dans l'exemple 1, le peroxyde d'hydrogène utilisé est à 2,7 % en poids par rapport au poids total de la composition. Dans l'exemple 2, le peroxyde d'hydrogène utilisé est à 6% en poids par rapport au poids total de la composition. Le pH des compositions de peroxyde d'hydrogène est de l'ordre de 2,3.

On obtient ainsi deux compositions tinctoriales prêtes à l'emploi.

La concentration de la monoéthanolamine dans les compositions prête à l'emploi des exemples 1 et 2 sont respectivement de 2,5 % et de 4 % en poids, par rapport au poids total de la composition prête à l'emploi.

Le pH final de ces deux compositions tinctoriales sont respectivement de 9,8 et de 10.

Après l'obtention de ces compositions tinctoriales prêtes à l'emploi, on a appliqué ces deux compositions sur des cheveux gris à 90 % de blancs et laissé poser pendant 20 minutes.

Les cheveux ont ensuite été lavés avec un shampooing standard puis rincés à l'eau et séchés.

La coloration des cheveux a été évaluée de manière visuelle.

Il est rappelé que la notion de « ton » repose sur la classification des nuances naturelles, un ton séparant chaque nuance de celle qui suit ou la précède immédiatement. Cette définition et la classification des nuances naturelles sont bien connues des professionnels de la coiffure et publiée dans l'ouvrage « Sciences des traitements capillaires » de Charles ZVIAK 1988, Ed.Masson, pp.215 et 278.

| 90 % Cheveux blancs naturels | Hauteur de ton | Reflet |
|---|---|---|
| Composition 1 | Châtain | Naturel |
| Composition 2 | Blond foncé | Cuivré acajou |

On ne constate pas d'inconfort lié à l'application sur les cheveux des deux compositions tinctoriales prêtes à l'emploi selon l'invention.

En particulier, on observe que l'application de ces deux compositions ne conduit pas à des irritations sur le cuir chevelu.

En outre ces compositions tinctoriales permettent d'obtenir des colorations qui sont notamment puissantes, chromatiques, peu sélectives et qui résistent bien aux shampooings.

### Exemple II

On a préparé une composition (A) conforme à l'invention et une composition (B) ne comprenant pas de silicone aminée à partir des composés indiqués dans le tableau ci-dessous :
(quantité exprimée en grammes sauf autre indication)

| | A (invention) | B (comparatif) |
|---|---|---|
| DC2-8299 de la société DOW CORNING (silicone aminée selon l'invention) | 3 MA | - |
| Acide laurique | 3 | 3 |
| Alcool laurique oxyéthyléné (12 OE) | 7 | 7 |
| Alcool décylique oxyéthyléné (3 OE) | 10 | 10 |
| Alcool cétylstéarylique (C16-18 50/50) | 11,5 | 11,5 |
| Alcool oléocétylique oxyéthyléné (30 OE) | 4 | 4 |
| Polycondensat tétraméthyl hexamethylènediamine / dichloro 1,3-propylène en solution aqueuse à 60% | 2 | 2 |
| Chlorure de poly diméthyl diallyl ammonium dans l'eau à 40% | 6 | 6 |
| Polymère carboxyvinylique | 0,4 | 0,4 |
| Propylène glycol | 10 | 10 |
| Monoéthanolamine pure | 5,45 | 5,45 |
| Silice pyrogénée | 1,2 | 1,2 |
| 1-méthyl 2,5-diamino benzène | 0,04 | 0,04 |
| 1-méthyl 2-hydroxy 4-béta-hydroxyéthylamino benzène | 0,096 | 0,096 |
| 1,3-dihydroxybenzène | 0,07 | 0,07 |
| 1-hydroxy 3-amino benzène | 0,026 | 0,026 |
| 1-hydroxy 4-amino benzène | 0,41 | 0,41 |
| Réducteur, séquestrant, antioxydant, parfum, opacifiants | Qs | Qs |
| Eau | Qs 100 | Qs 100 |

On a mélangé une partie de chacune de ces compositions, avec une partie et demie de peroxyde d'hydrogène. Dans ces compositions, le peroxyde d'hydrogène utilisé est à 2,7% en poids par rapport au poids total de la composition. Le pH des compositions de peroxyde d'hydrogène est de l'ordre de 2,3.

Sur 5 modèles (femmes âgés de 44 à 60 ans) ayant un cuir chevelu particulièrement réactif aux produits de coloration, sont appliquées en comparatif d'un côté la composition (B) et de l'autre côté de la composition (A) selon l'invention. Après 20 minutes de pause et sans rinçage intermédiaire, les cheveux sont rincés, lavés avec un shampooing standard et rincés à nouveau.

Interrogées sur l'intensité de leurs sensations durant le temps de pause de la coloration, les cinq modèles notent sur une échelle 0 (aucune sensation d'inconfort) à 10 (inconfort intolérable), l'inconfort ressenti au niveau du cuir chevelu. Les moyennes des notes obtenues sont indiquées dans le tableau ci-dessus :

| | Avec la composition (A) | Avec la composition (B) |
|---|---|---|
| Inconfort | 1,7 | 3,5 |

On constate une diminution de 50% de l'inconfort avec la composition A contenant la silicone aminée selon l'invention, par rapport à la composition B ne contenant pas cette silicone.

## Revendications

1. Composition pour la teinture des fibres kératiniques, **caractérisée par le fait qu'**elle comprend, dans un milieu approprié pour la teinture :
- au moins une base d'oxydation,
- au moins un coupleur,
- au moins une silicone aminée de masse moléculaire en poids Mw supérieure ou égale à 75 000, et
- au moins de la monoéthanolamine présente en une quantité allant de 1 à 10% en poids, par rapport au poids total de la composition, et
- au moins un agent oxydant.

2. Composition selon la revendication 1, **caractérisée par le fait que** ladite silicone aminée est de formule (I) suivante : 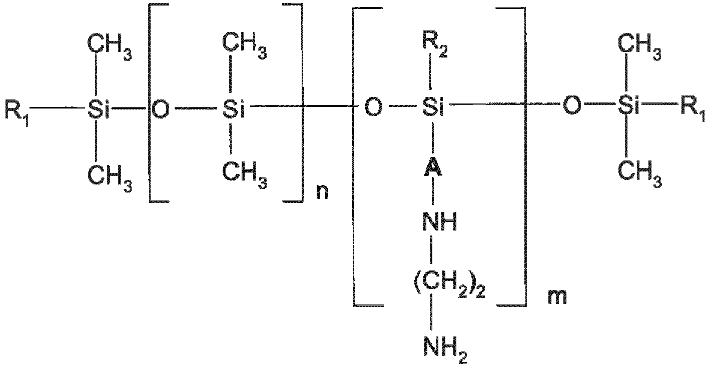 dans laquelle :
A désigne un radical alkylène linéaire ou ramifié en C₂-C₈,
m et n sont des nombres tels que la masse moléculaire en poids Mw est supérieure ou égale à 75 000,
R₁, R₂ désignent indépendamment l'un de l'autre un radical alkyle en C₁-C₄ ou un radical alcoxy en C₁-C₄ ou un radical hydroxy.

3. Composition selon la revendication 2, **caractérisée par le fait que** A désigne un radical alkylène linéaire ou ramifié en C₃.

4. Composition selon la revendication 2 **caractérisée en ce que** le radical alkyle de R₁ et/ou R₂ est un radical méthyle et que le radical alcoxy de R₁ et/ou R₂ est un radical méthoxy.

5. Composition selon l'une quelconque des revendications précédentes, **caractérisée par le fait que** la viscosité de ladite silicone aminée est supérieure à 25 000 mm²/s à 25°C.

6. Composition selon l'une quelconque des revendications précédentes, **caractérisée par le fait que** ladite silicone aminée présente une masse moléculaire moyenne en poids allant de 75 000 à 1000000 et de préférence allant de 100 000 à 200000.

7. Composition selon l'une quelconque des revendications précédentes **caractérisée en ce que** la charge cationique de la silicone aminée est inférieure ou égale à 0,06 meq/g.

8. Composition selon l'une quelconque des revendications précédentes, **caractérisée par le fait que** la ou les bases d'oxydation sont choisies parmi les ortho- ou para- phénylènediamines, les bases doubles, les ortho- ou para- aminophénols, et les bases hétérocycliques, ainsi que les sels d'addition de ces composés avec un acide.

9. Composition selon l'une quelconque des revendications précédentes, **caractérisée par le fait qu'**elle comprend en outre du métasilicate de sodium.

10. Composition selon l'une quelconque des revendications précédentes **caractérisée par le fait qu'**elle présente un pH allant de 3 à 12 et de préférence de 7 à 11.

11. Procédé de teinture des fibres kératiniques **caractérisée par le fait qu'**il consiste à appliquer sur lesdites fibres une composition telle que définie selon l'une quelconque des revendications précédentes, pendant un temps suffisant pour développer la coloration désirée.

12. Procédé de teinture selon la revendication 11, **caractérisée par le fait que** l'agent oxydant est choisi parmi le peroxyde d'hydrogène, le peroxyde d'urée, les bromates ou ferricyanures de métaux alcalins, les persels, les enzymes d'oxydoréduction avec éventuellement leur donneur ou cofacteur respectif.

13. Utilisation d'une composition telle que définie selon l'une quelconque des revendications 1 à 10 pour la coloration des fibres kératiniques, en particulier des fibres kératiniques humaines telles que les cheveux.

## Patentansprüche

1. Zusammensetzung zum Färben von Keratinfasern, **dadurch gekennzeichnet, dass** sie in einem für die Färbung geeigneten Medium Folgendes umfasst:
- mindestens eine Oxidationsbase,
- mindestens einen Kuppler,
- mindestens ein Aminosilikon mit einem gewichtsmittleren Molekulargewicht Mw größer gleich 75.000 und
- mindestens Monoethanolamin, das in einer Menge im Bereich von 1 bis 10 Gew.-%, bezogen auf das Gesamtgewicht der Zusammensetzung, vorliegt, und
- mindestens ein Oxidationsmittel.

2. Zusammensetzung nach Anspruch 1, **dadurch gekennzeichnet, dass** das Aminosilikon die folgende Formel (I) aufweist: 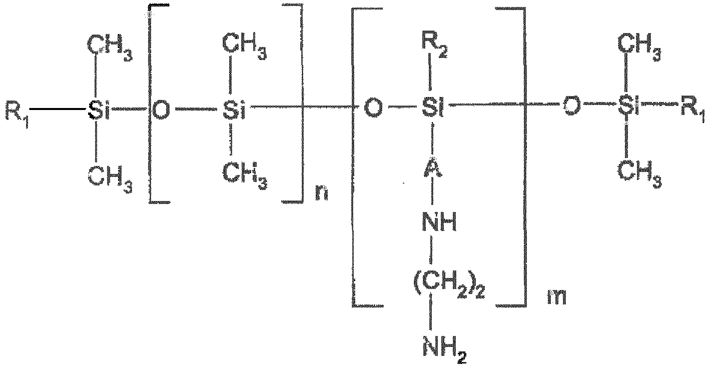 worin:
A für einen linearen oder verzweigten C₂-C₈-Alkylenrest steht,
m und n solche Zahlen sind, dass das gewichtsmittlere Molekulargewicht Mw größer gleich 75.000 ist,
R₁ und R₂ unabhängig voneinander für einen C₁-C₄-Alkylrest oder einen C₁-C₄-Alkoxyrest oder einen Hydroxyrest stehen.

3. Zusammensetzung nach Anspruch 2, **dadurch gekennzeichnet, dass** A für einen linearen oder verzweigten C₃-Alkylenrest steht.

4. Zusammensetzung nach Anspruch 2, **dadurch gekennzeichnet, dass** es sich bei dem Alkylrest von R₁ und/oder R₂ um einen Methylrest handelt und es sich bei dem Alkoxyrest von R₁ und/oder R₂ um einen Methoxyrest handelt.

5. Zusammensetzung nach einem der vorhergehenden Ansprüche, **dadurch gekennzeichnet, dass** die Viskosität des Aminosilikons größer als 25.000 mm²/s bei 25°C ist.

6. Zusammensetzung nach einem der vorhergehenden Ansprüche, **dadurch gekennzeichnet, dass** das Aminosilikon ein gewichtsmittleres Molekulargewicht im Bereich von 75.000 bis 1.000.000 und vorzugsweise im Bereich von 100.000 bis 200.000 aufweist.

7. Zusammensetzung nach einem der vorhergehenden Ansprüche, **dadurch gekennzeichnet, dass** die kationische Ladung des Aminosilikons kleiner gleich 0,06 meq/g ist.

8. Zusammensetzung nach einem der vorhergehenden Ansprüche, **dadurch gekennzeichnet, dass** die Oxidationsbase bzw. die Oxidationsbasen aus ortho- oder para-Phenylendiaminen, Doppelbasen, ortho- oder para-Aminophenolen und heterocyclischen Basen sowie Additionssalzen dieser Verbindungen mit einer Säure ausgewählt ist bzw. sind.

9. Zusammensetzung nach einem der vorhergehenden Ansprüche, **dadurch gekennzeichnet, dass** sie außerdem Natriummetasilikat umfasst.

10. Zusammensetzung nach einem der vorhergehenden Ansprüche, **dadurch gekennzeichnet, dass** sie einen pH-Wert im Bereich von 3 bis 12 und vorzugsweise von 7 bis 11 aufweist.

11. Verfahren zum Färben von Keratinfasern, **dadurch gekennzeichnet, dass** es darin besteht, dass man auf die Fasern eine Zusammensetzung gemäß einem der vorhergehenden Ansprüche über einen zur Entwicklung der gewünschten Färbung ausreichenden Zeitraum aufbringt.

12. Färbeverfahren nach Anspruch 11, **dadurch gekennzeichnet, dass** das Oxidationsmittel aus Wasserstoffperoxid, Harnstoffperoxid, Alkalimetallbromaten oder -ferricyaniden, Persalzen und Redoxenzymen, gegebenenfalls in Gegenwart ihres jeweiligen Donors oder Cofaktors, ausgewählt wird.

13. Verwendung einer Zusammensetzung gemäß einem der Ansprüche 1 bis 10 zur Färbung von Keratinfasern, insbesondere menschlichen Keratinfasern wie den Haaren.

## Claims

1. Composition for the dyeing of keratinous fibres, **characterized in that** it comprises, in a medium appropriate for dyeing:
- at least one oxidation base,
- at least one coupler,
- at least one aminated silicone with a weight-average molecular weight M_{w} of greater than or equal to 75 000, and
- at least monoethanolamine present in an amount ranging from 1 to 10% by weight, with respect to the total weight of the composition, and
- at least one oxidizing agent.

2. Composition according to Claim 1, **characterized in that** the said aminated silicone is of following formula (I): 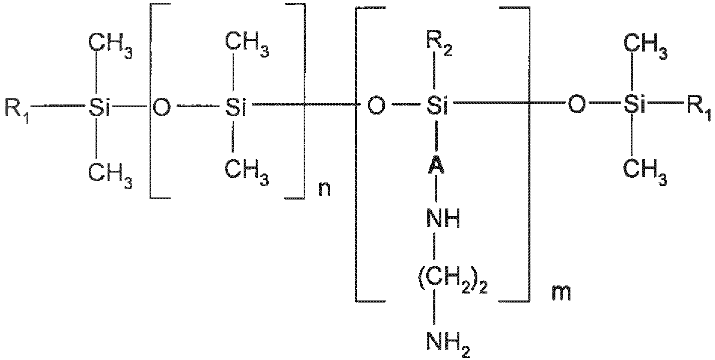 in which:
A denotes a linear or branched C₂-C₈ alkylene radical,
m and n are numbers such that the weight-average molecular weight Mw is greater than or equal to 75 000,
R₁ and R₂ denote, independently of one another, a C₁-C₄ alkyl radical or a C₁-C₄ alkoxy radical or a hydroxyl radical.

3. Composition according to Claim 2, **characterized in that** A denotes a linear or branched C₃ alkylene radical.

4. Composition according to Claim 2, **characterized in that** the alkyl radical of R₁ and/or R₂ is a methyl radical and **in that** the alkoxy radical of R₁ and/or R₂ is a methoxy radical.

5. Composition according to any one of the preceding claims, **characterized in that** the viscosity of the said aminated silicone is greater than 25 000 mm²/s at 25°C.

6. Composition according to any one of the preceding claims, **characterized in that** the said aminated silicone exhibits a weight-average molecular weight ranging from 75 000 to 1 000 000 and preferably ranging from 100 000 to 200 000.

7. Composition according to any one of the preceding claims, **characterized in that** the cationic charge of the aminated silicone is less than or equal to 0.06 meq/g.

8. Composition according to any one of the preceding claims, **characterized in that** the oxidation base or bases are chosen from ortho- or para-phenylenediamines, double bases, ortho- or para-aminophenols, heterocyclic bases and the addition salts of these compounds with an acid.

9. Composition according to any one of the preceding claims, **characterized in that** it additionally comprises sodium metasilicate.

10. Composition according to any one of the preceding claims, **characterized in that** it exhibits a pH ranging from 3 to 12 and preferably from 7 to 11.

11. Method for dyeing keratinous fibres, **characterized in that** it consists in applying, to the said fibres, a composition as defined according to any one of the preceding claims for a time sufficient to develop the desired colouring.

12. Dyeing method according to Claim 11, **characterized in that** the oxidizing agent is chosen from hydrogen peroxide, urea hydrogen peroxide, alkali metal bromates or ferricyanides, persalts, oxidoreduction enzymes with optionally their respective donor or cofactor.

13. Use of a composition as defined according to any one of Claims 1 to 10 for dyeing keratinous fibres, in particular human keratinous fibres, such as the hair.
